# EUROPEAN PATENT APPLICATION

(11) **EP 1 393 676 A1**
(43) Date of publication of application: **03.03.2004**
(21) Application number: 03254806.7
(22) Date of filing: 31.07.2003
(51) Int. Cl.: A61B 5/097, F16K 15/14, G01N 33/497

(54) **Sealable air sampling bag and method for using the same**

(30) Priority: 23.08.2002 US 227177
(71) Applicant: Hamilton Enterprises Inc. d/b/a Quintron Instrument Company, Milwaukee, Wisconsin 53215 (US)
(72) Inventor: Hamilton, Steven D., Greenfield, Wisconsin 53221 (US)
(74) Representative: Dee, Ian Mark

(57) **Abstract**

An air sampling device and method is described, the air sampling device having a bag (10), an interior (99) of the bag, a conduit (20) coupled with the interior of the bag, the conduit generally being a duck-bill valve within the interior of the bag. A breath intake structure (100), for example a staw, is communicatively coupled with the interior of the bag through the conduit during use.

## Description

### Related Application

This application is a continuation-in-part of co-pending patent application Serial No. 09/497,972 filed 2 February 2000, and entitled Sealable Air Sampling Bag.

### Background of the Invention

This invention relates to the field of sampling air from the lungs and specifically to the field of obtaining a sample of a person's air, including alveolar air from the alveoli of the lungs of a person.

Air from the lungs of a person can be used for many different types of testing that would otherwise require the person to undergo an invasive procedure. For example, alveolar air can be analyzed for, but not limited to, the noninvasive diagnosis of a wide variety of conditions including the noninvasive diagnosis of stomach infections related to a high incidence of ulcers, enzymatic deficiencies, and metabolic conditions and/or abnormalities. Crucial to any such testing is the ability to get an accurate sample containing a sufficient volume of air representative of true alveolar air, necessary for specific testing.

A simple to use, inexpensive, and user-friendly apparatus is desired to collect and store human breath samples. In order to collect true alveolar air, an apparatus smaller in potential volume than the volume of a human breath is desired, in order to purge air from the apparatus and subject to be tested that is not true alveolar air. One such apparatus is disclosed in U.S. Pat. 5,432,094 to Delente (July 11, 1995). Another such apparatus is disclosed in U.S. Pat. 5,327,901 to Delente (July 12, 1994).

It is desirable to have a sample container that is cost efficient, light weight, dependable and optionally capable of withstanding contraction and expansion common to high elevations, pressure changes, and temperature changes, such as within an airplane cargo department or car trunks, without bursting and losing the sample. Additionally, it is desirable to have a sample container that is easy to operate, without risk of sample contamination due to dilution, diffusion, or through interaction of the sample and container materials.

### Summary of the Invention

An air sampling device for sampling air received from the exhalation of breath of a person is disclosed. The air sampling device is comprised of a breath intake structure used to exhale a breath into an expandable bag structure through an inlet. The breath intake structure can be constructed of commonly available materials, and a drinking straw may be the preferred breath intake structure. The expandable bag structure is preferably constructed of a supple, airtight, gas impermeable, and inert material. The breath intake structure is selectively communicatively coupled with expandable bag structure through the inlet.

The air sampling device can be labeled with a sample identifier, such as possibly a barcode or machine-readable system. The sample identifier provides a convenient method for sequentially labeling air samples, or should the samples include a preloaded dessicant or a chemical indicator, or sample conditioner, should samples from different patients be shipped together.

An example of a conditioner is a drying agent. A conditioner may or may not comprise a dessicant. Another example of a conditioner is an alkaline substance that absorbs water or CO2 to preserve ammonia.

In another embodiment of the present invention, the air sampling device can include an outer shell encapsulating the expandable bag structure. The outer shell can capture the air sample should the expandable bag structure rupture. The outer shell has an outer shell inlet to the expandable bag structure interior providing a conduit for the breath intake structure. In this embodiment, the breath intake structure is selectively communicatively coupled with the expandable bag structure interior between the one interior surface of the expandable bag structure inlet and the removable adhesive patch cover. In this embodiment, the adhesive inlet seal patch is applied to the exterior of the outer shell. Again, the adhesive inlet seal patch can include the sample identifier. The outer shell is sized to contain a volume greater than a volume of the inner expandable bag structure, so that if the air sample is depressurized and ruptures the inner expandable bag structure, the outer shell will still contain the air sample.

A method for using the air sampling device is also disclosed and is substantially as follows. The breath intake structure is inserted into the expandable bag structure inlet and a conduit. A patient places the breath intake structure into the mouth against his or her lips and exhales into it in a normal manner. During an initial phase of a patient's exhalation into the breath intake structure, the expired air flows into the initially empty expandable bag structure, filling the expandable bag structure. During the remaining phase of the patient's exhalation through the intake structure, the breath from the initial phase of the patient's exhalation is expelled through the inlet, and is replaced by alveolar air that remains in the expandable bag structure.

To seal the expandable bag structure, the breath intake structure is removed and the bag is sealed.

Next, to further seal the expandable bag structure if desired, the adhesive inlet seal patch is separated from the adhesive inlet seal patch cover, and the adhesive inlet seal patch is applied to the expandable bag structure, further sealing the inlet. The sample identifier can be either written or pre-labeled on the adhesive inlet seal patch or outer shell.

If an outer shell is employed, the same sampling methodology is used, with the exception that following the step of pressing together the exterior surfaces of the inlet with the adhesive patch, the adhesive inlet seal patch is separated from the adhesive inlet seal patch cover, and the adhesive inlet seal patch is applied to the outer shell, covering the outer shell inlet.

If the expandable bag structure bursts, the outer shell will still contain the air sample. Accordingly, when a person exhales into the breath intake structure, a first predetermined volume of breath enters the expandable bag structure until the expandable bag structure is expanded to its predetermined size Next, the remainder of the breath, which includes alveolar air from the person's lungs, enters and expands the expandable bag structure and forces the waste air from the expandable bag structure. This results in the expandable bag being filled with a sample of alveolar air.

### Description of the Drawings

Figure 1 is a top view of a bag and a breath intake structure.
Figure 2 is a top view of a bag and a breath intake structure inserted into the bag through a conduit.
Figure 3 is a side cross sectional view of a bag and a breath intake structure.
Figure 4 is a side cross sectional view of a bag and a breath intake structure being inserted into the bag through a conduit.
Figure 5 is a side cross sectional view of a bag and a breath intake structure inserted into the bag through a conduit.
Figure 6 is a side cross sectional view of a bag and a breath intake structure inserted into the bag through a conduit, the bag receiving air through the breath intake structure.
Figure 7 is a side cross sectional view of a bag and a breath intake structure being withdrawn from the bag through a conduit.
Figure 8 is a side cross sectional view of a bag filled with air and a conduit.
Figure 9 is a perspective view of a breath intake structure and a conduit.
Figure 10a is a side perspective view of an alternate embodiment of an air sampling device, a bag having an outer shell encapsulating the bag.
Figure 10b is a side perspective view of an air sampling device including an outer shell, and the bag bursting.
Figure 10C is a side perspective view of an air sampling device including an outer shell, the bag burst, and the outer shell capturing the breath sample.

### Detailed Description

Although the disclosure hereof is detailed and exact to enable those skilled in the art to practice the invention, the physical embodiments herein disclosed merely exemplify the invention which may be embodied in other specific structure. While the preferred embodiment has been described, the details may be changed without departing from the invention, which is defined by the claims.

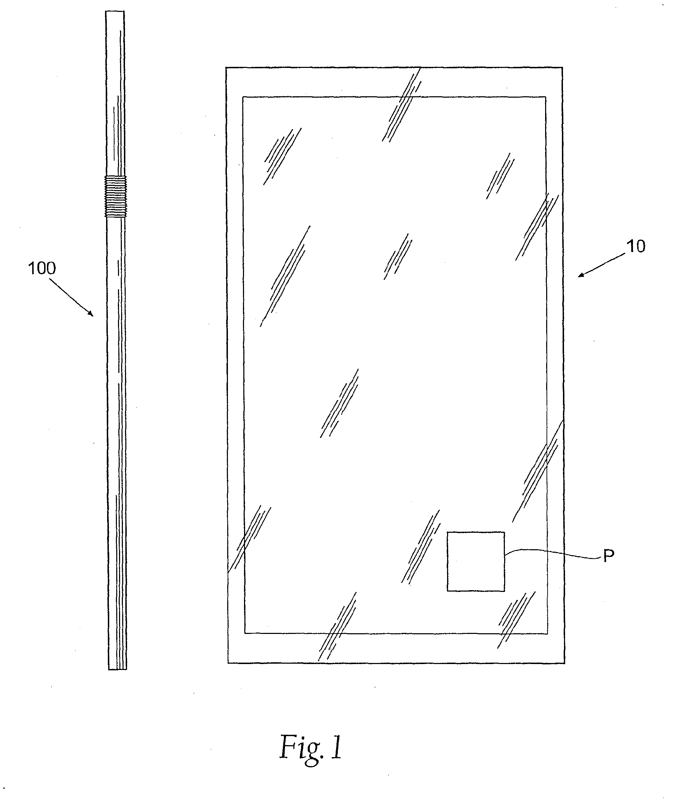

Referring to Fig. 1, a top view of a bag 10 and a breath intake structure 100 is shown. The breath intake structure 100 as pictured comprises an ordinary drinking straw, although the structure or type of breath intake structure 100 can be modified in any way.

The bag 10 comprises an interior 99 of the bag 10 for filling with air, which will be described later. The bag 10 also comprises an opening (described later) through which the breath intake structure is inserted.

In a preferred embodiment, a patch P is provided on the exterior of the bag 10. The patch P is a preferable location for an operator to insert a needle to withdraw an air sample within a laboratory (not shown). The patch P is provided to prevent the bag from tearing when the needle (not shown) is inserted. It has been found that without a patch P on the bag 10, the bag 10 is susceptible to tearing, potentially losing the air sample. The patch 10 can be located anywhere on the bag 10. Alternatively, although not preferably, the patch P can be provided on an interior of the bag 10 and then an indicator marker (not shown) could be provided on the visible portion of the bag 10 to target the operator's insertion of the needle.

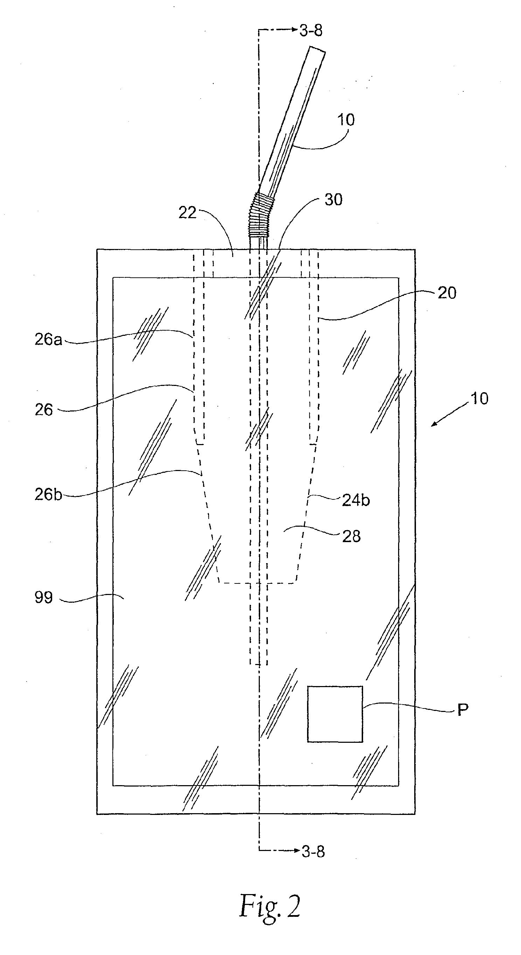

Referring to Fig. 2, a top view of the bag 10 and the breath intake structure 100 inserted into the bag 10 through an inlet 30 and a conduit 20 is shown. The breath intake structure 100 is selectively communicatively coupled with expandable bag structure interior 99 through the inlet 30.

It is preferable, though not required, that the bag 10 is of two ply construction, the two plies sealed together, such that the inlet 30 is formed at a location where the plies are left unsealed. Of course, more or less plies may be used in the bag construction. The bag structure 10 is preferably expandable, and constructed of a supple, inert, and airtight material. One such supple and airtight material that performs suitably is thin polyester film, but foil laminate and a variety of other materials could also perform suitably. It should be easily recognizable to one skilled in the art that any number of materials other than foil laminate can be used for the bag structure 10. Other examples of materials that could be used for constructing the present invention include for illustration, but not by way of limitation: Tedlar® , Saranex® , Saran® , and Teflon® . Those skilled in the art will appreciate that the material can vary widely based on the characteristics of the gases desired to be sampled. The materials chosen should be inert and exhibit relative impermeability to the gases desired to sample, and any materials chosen that are relatively permeable to the gases desired to sample would not be preferred.

The conduit 20, is shown carried by the bag 10. It is preferable that the conduit 20 has a conduit inlet 22 that receives the breath intake structure 100 when in use. The conduit 20 can be coupled with the bag 10 within the bag inlet 30.

As shown, it is preferred that the conduit 20 is formed of two plies, a first overlying ply 24 and a second underlying ply 26, of which only one ply is visible from this view. For the purpose of illustration, the structure of the conduit 20 will be described here with relation to the second underlying ply 26, it being understood that preferably the plies 24 and 26 are substantially similar.

The first ply 24 and second ply 26 are coupled along a first length 24a and a second length 26a. The first ply 24 and second ply 26 are not coupled along a first length 24b and a second length 26b, the uncoupled lengths 24b and 26b being at a distal end 28 of the conduit 20. The result of the uncoupled lengths 24b and 26b of the plies 24 and 26 is that two flaps are formed at the distal end 28 of the conduit 28, one flap being formed from each ply 24 and 26. I will refer to the two flaps as forming a duck-bill valve.

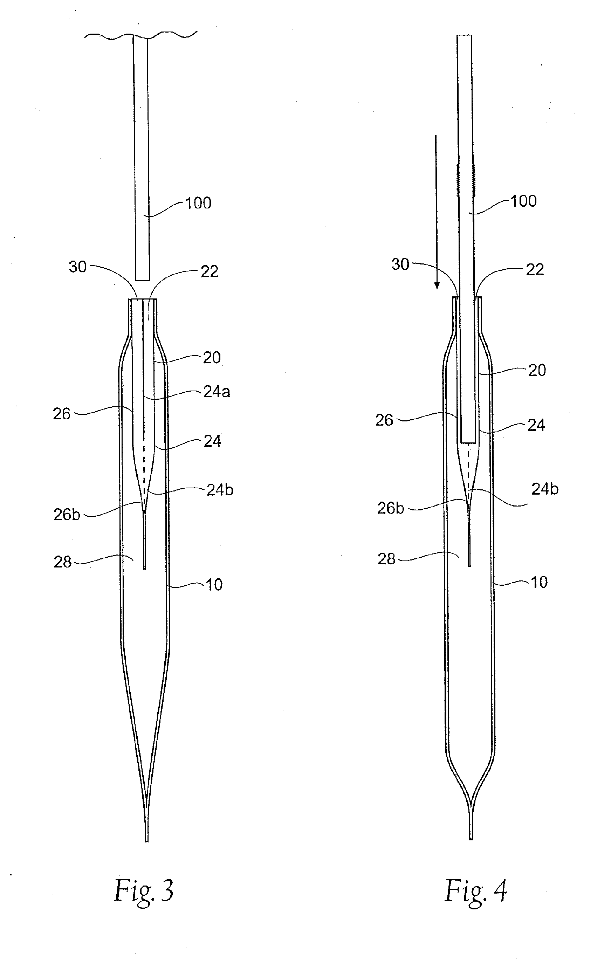

Referring to Fig. 3 a side cross sectional view of the bag 10 and breath intake structure 100 is shown. As shown, the breath intake structure 100 is prepared for insertion into the bag 10 through conduit 20 inlet 22, in addition to bag inlet 30.

Referring to Fig. 4 a side cross sectional view of the bag 10 and the breath intake structure 100 being inserted into the bag 10 through the conduit 20 is shown. As shown, the breath intake structure 100 is being inserted into the bag 10 through conduit 20 inlet 22, in addition to bag inlet 30.

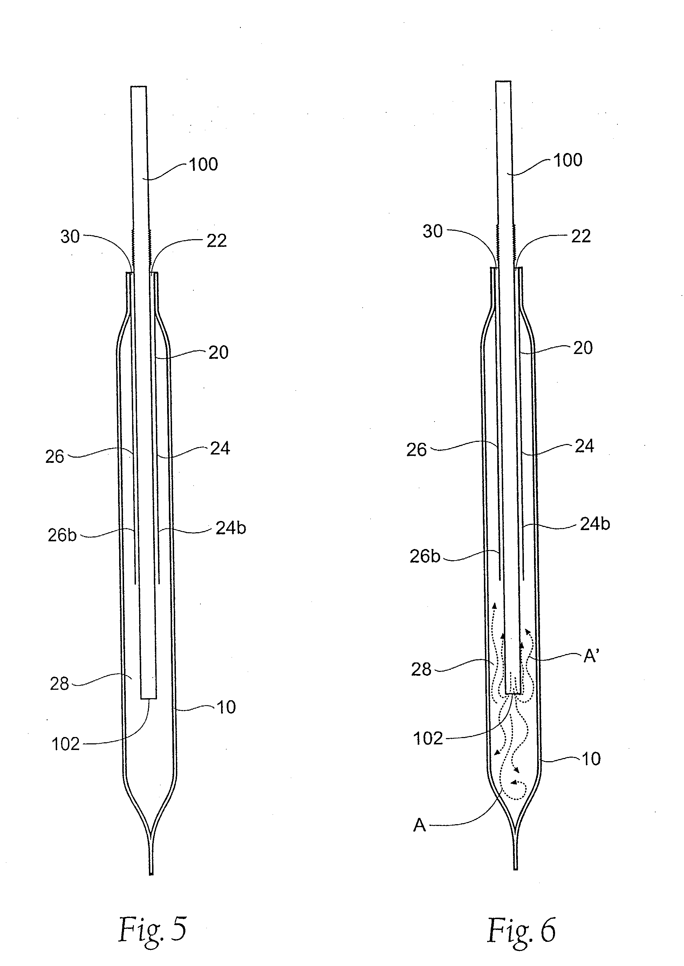

Referring to Fig. 5 a side cross sectional view of the bag 10 and the breath intake structure 100 inserted into the bag 10 through the conduit 20 is shown. As shown, the breath intake structure 100 is inserted into the bag 10 through conduit 20 inlet 22, in addition to bag inlet 30. An outer end 102 of the breath intake structure 100 is located past the distal end 28 of the conduit 20.

Referring to Fig. 6 a side cross sectional view of the bag 10 and the breath intake structure 100 inserted into the bag 10 through the conduit 20, the bag receiving air through the breath intake structure is shown. As shown, the breath intake structure 100 is inserted into the bag 10 through conduit 20 inlet 22, in addition to bag inlet 30. An outer end 102 of the breath intake structure 100 is located past the distal end 28 of the conduit 20. In Fig. 6, a user (not shown) is blowing air A into the conduit 20, the air A being received by the bag 10. Air A is blown into the bag 10 until a breath is exhausted. It is desired that a first portion of the breath A' exit the bag 10 back through the conduit 20, in order that the air sample reflect true alveolar air.

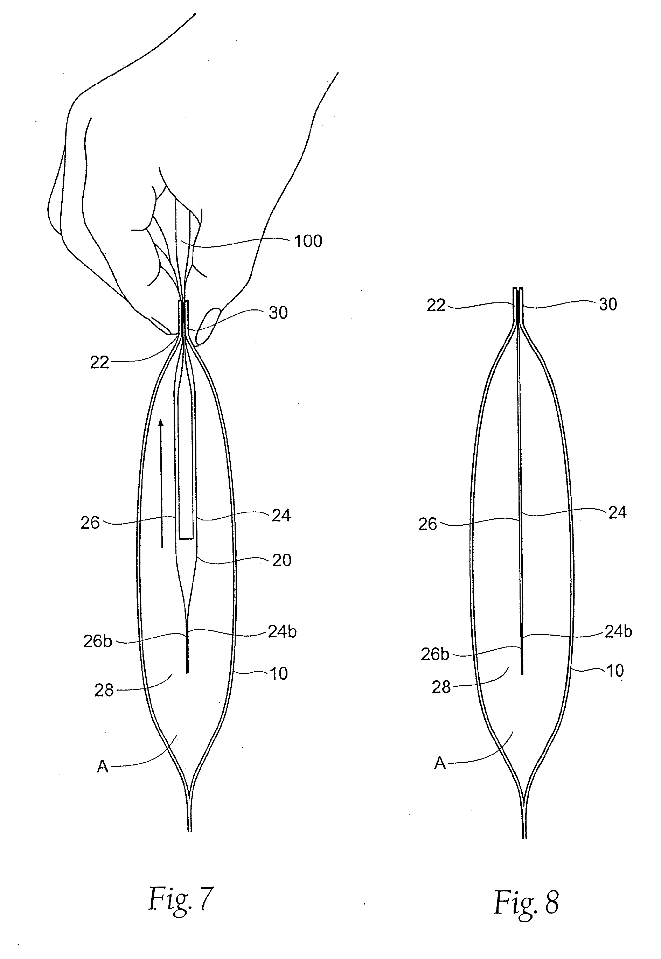

Referring to Fig. 7 a side cross sectional view of the bag 10 and the breath intake structure 100 being withdrawn from the bag 10 through the conduit 20 is shown. After the breath from a user has been fully exhaled through the breath intake structure 100 and into the bag 10, it is desired to seal the bag 10 for laboratory analysis of the air A contained therein. To withdraw the breath intake structure 100 from the bag 10, a user may pinch the conduit inlet 22 and the bag inlet 30 as shown. When the breath intake structure 100 is withdrawn, the breath intake structure 100 compresses under the influence of the pinching, in order that all air is removed from the breath intake structure 100 and no exterior air may enter the bag 10.

Following the withdrawal of the breath intake structure 100, the bag 10, and particularly the conduit inlet 22 and/or bag inlet 30 can be sealed in any fashion. It is preferable to apply an adhesive patch (not shown) over the conduit inlet 22 and/or bag inlet 30 to seal the bag.

Referring to Fig. 8 a side cross sectional view of the bag filled with air A and the conduit 20 is shown. In this condition, the bag 10 and the air A contained therein is ready for transport, processing, and laboratory sampling in accordance with desired objectives.

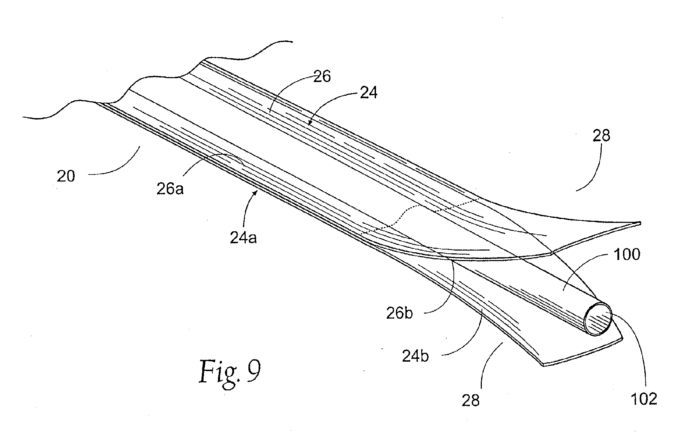

Referring to Fig. 9 a perspective view of the breath intake structure 100 and the conduit 20 is shown. The first ply 24 and second ply 26 are coupled along a first length 24a and a second length 26a. The first ply 24 and second ply 26 are not coupled along a first length 24b and a second length 26b, the uncoupled lengths 24b and 26b being at a distal end 28 of the conduit 20. The result of the uncoupled lengths 24b and 26b of the plies 24 and 26 is that two flaps are formed at the distal end 28 of the conduit 28, one flap being formed from each ply 24 and 26.

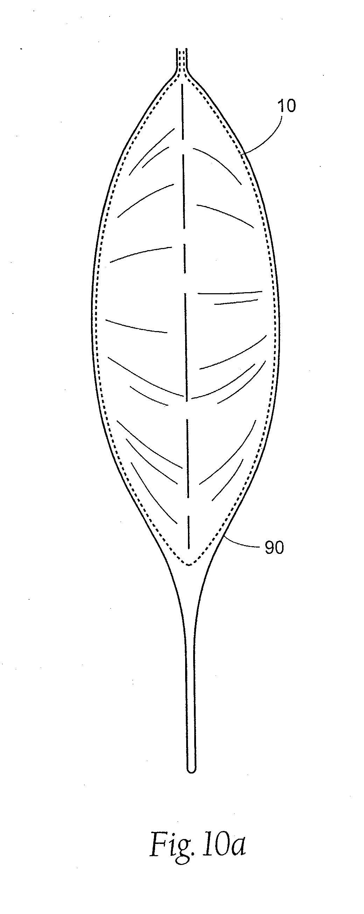

In another embodiment of the present invention, the air sampling device can include an outer shell 90 encapsulating the bag 10. If the outer shell 90 is used, the bag 10 is filled as shown in Figure 10A.

The outer shell 90 can capture the air sample should the bag 10 structure rupture. The outer shell 90 has an outer shell inlet to the bag 10 interior providing a conduit for the breath intake structure 100 (not shown on Figs. 10A -C). In this embodiment, the breath intake structure is selectively communicatively coupled with the interior of the bag 10. The outer shell 90 is sized to contain a volume greater than a volume of the bag 10 so that if the air sample is depressurized and ruptures the bag 10, the outer shell 90 will still contain the air sample. The outer shell 90 is sealed shut, as is the bag 10.

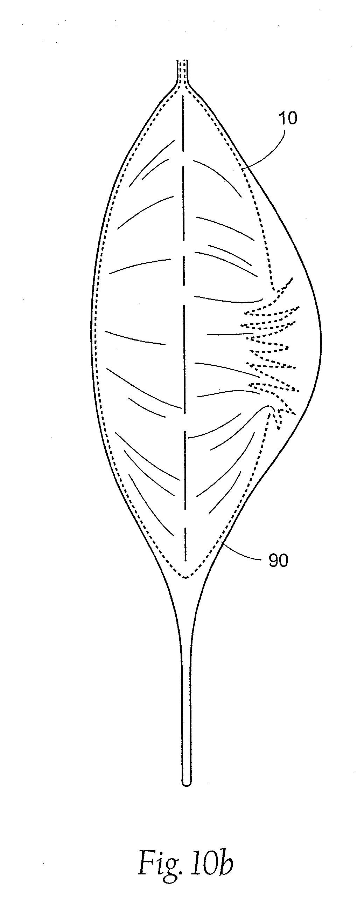

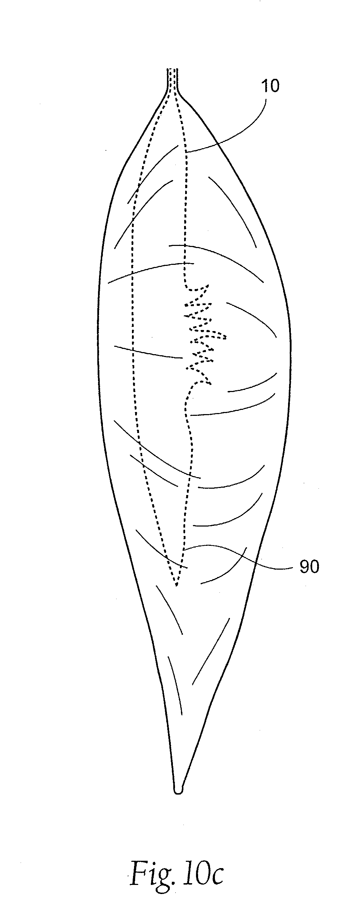

Next, if the bag 10 bursts, as shown in Figure 10B, the outer shell 90 will still contain the air sample, as shown in Figure 10C. This feature is particularly useful if the samples must be transported by airplane, exposing the samples to wide pressure and temperature variations. It is noted that if an outer shell 90 is used, the patch P can be applied to the outer shell 90 (not shown).

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

The above described embodiments of this invention are merely descriptive of its principles and are not to be limited. The scope of this invention instead shall be determined from the scope of the following claims, including their equivalents.

## Claims

1. An air sampling device comprising:
a bag;
an interior of the bag;
a conduit coupled with the interior of the bag;
said conduit comprising a duck-bill valve within the interior of the bag.

2. An air sampling device according to claim 1, the device further comprising a breath intake structure communicatively coupled with the interior of the bag.

3. An air sampling device according to claim 2, wherein the breath intake structure comprises a straw.

4. An air sampling device according to claim 2, wherein the breath intake structure is communicatively coupled with the interior of the bag through the conduit.

5. An air sampling device according to claim 1, wherein the bag is expandable.

6. An air sampling device according to claim 1, the bag further comprising a dessicant contained within the bag.

7. An air sampling device according to claim 1, the bag further comprising a chemical indicator contained within the bag.

8. An air sampling device according to claim 1, the bag further comprising a conditioner contained within the bag.

9. An air sampling device according to claim 1, the bag further comprising a sample identifier on an outside of the bag.

10. An air sampling device according to claim 9, wherein the sample identifier comprises a machine readable code.

11. An air sampling device according to claim 1, the bag further comprising an outer shell containing said bag.

12. An air sampling device according to claim 11, where said outer shell captures air contained within said bag if said bag bursts.

13. An air sampling device according to claim 11, said air sampling device further comprising a patch coupled with said outer shell.

14. An air sampling device according to claim 13, wherein said patch comprises rubber.

15. An air sampling device according to claim 13, wherein said patch is applied to the exterior of said outer shell.

16. An air sampling device according to claim 1, the said air sampling device further comprising a patch coupled with said bag.

17. An air sampling device according to claim 16, wherein said patch comprises rubber.

18. An air sampling device according to claim 16, wherein said patch is applied to the exterior of said bag.

19. An air sampling device for sampling air received from the exhalation of breath of a person, said air sampling device comprising:
a breath intake structure;
an expandable bag structure having an interior;
an inlet in the expandable bag structure to the expandable bag structure interior;
a conduit coupled with the inlet, the conduit comprising a first and a second ply of material, each ply having a first and a second edge;
said first and said second edges coupled together along a length of said edges.

20. An air sampling device according to claim 19, wherein said length of said edges is less than an entire length of said edges.

21. An air sampling device according to claim 19, wherein said edges are coupled along a portion of said edges.

22. A method of sampling alveolar air comprising:
inserting a breath intake structure into a bag having an inlet, a conduit between an interior and an exterior of the bag, and a duck-bill valve;
breathing a breath through the breath intake structure;
expelling a first portion of the breath through the conduit and out of the duck-bill valve to the exterior of the bag;
pinching the inlet;
withdrawing the breath intake structure.

23. A method according to claim 22, the method further comprising, after the step of withdrawing the breath intake structure, applying a seal to the conduit.
